# EUROPEAN PATENT APPLICATION

(11) **EP 1 224 937 A1**
(43) Date of publication of application: **24.07.2002**
(21) Application number: 00969952.1
(22) Date of filing: 20.10.2000
(51) Int. Cl.: A61K 33/18, A61K 47/48, A61K 47/38, A61K 47/42, A61K 47/26, A61P 17/02

(54) **STABLE PREPARATIONS FOR TREATING BEDSORE, SKIN ULCER AND WOUND**

(30) Priority: 22.10.1999 JP 30033099
(71) Applicant: NIPPI, INCORPORATED, Adachi-ku, Tokyo 120-0044 (JP)
(72) Inventor: HARA, Susumu, Arakawa-ku, Tokyo 116-0013 (JP); EBIHARA, Tetsuya, Adachi-ku, Tokyo 123-0873 (JP); KOYAMA, Yohichi, Kasumigauramachi, Niihari-gun, Ibaraki 3 (JP); NISHIZAWA, Masaru, Funabashi-shi, Chiba 273-0031 (JP); IRIE, Shinkichi, Yokohama-shi, Kanagawa 227-0033 (JP); SATO, Toshiaki, Kita-ku, Tokyo 115-0055 (JP); TANAKA, Yoshihiro, Matsudo-shi, Chiba 270-0021 (JP); TAKIGAWA, Tomoaki, Iwatsuki-shi, Saitama 339-0003 (JP); YOSHIDA, Satoshi, Kawagoe-shi, Saitama 350-1165 (JP); MIZUNO, Keizo, Tokorozawa-shi, Saitama 359-1152 (JP)
(74) Representative: Colmer, Stephen Gary
(86) International application number: JP0007335
(87) International publication number: WO0128571

(57) **Abstract**

The present invention provides a formulation for use in the treatment of decubitus, skin ulcer and wound, having higher stability and safety. The formulation is prepared by mixing a gelatin in which a consumption of iodine is not more than 15 mg/g as measured at pH 4.5 with a sugar and an iodophor.

## Description

### FIELD OF THE INVENTION

The present invention relates to a formulation for use in the treatment of decubitus, skin ulcer and wound, and particularly relates to the formulation for use in the treatment of decubitus, skin ulcer and wound, which is useful in the treatment of bedsore or the like.

### PRIOR ART

Hitherto, as one example of the formulations for use in the treatment of decubitus, skin ulcer and wound, it is known to prepare and administer the formulation in which sucrose and povidone-iodine were mixed as an agent used in a hospital. However, the formulation is unstable, and thus required to be prepared at the time when it is used. And also, a lot of time to prepare it is spended, and it was not appropriate for use and storage over a long period of time.

To improve those disadvatages there is proposed and actually employed such a technique as to prepare the ointment comprising sucrose and povidoneiodine together with water, a buffer and a certain polysaccharide (binder) (Japanese Patent Publication No. 32210/1989 B).

As examples of the publications which disclose the formulation for use in the treatment of decubitus and skin ulcer which comprises sucrose and povidoneiodine as effective components, in addition to said Japanese Patent Publication No. 32210/1989 B, there is well known an ointment in Japanese Patent Publication No. 40563/1997 A, and there is well known a powder in Japanese Patent Publications No. 12582/1996 A and No. 169655/1997 A.

In addition, in said Japanese Patent Publication No. 32210/1989 B it is described with comparative examples in which concrete data are shown that as regards albumin and gelatin among binders, even if pH was adjusted by buffer solution, they render the survival rate of effective iodine lower and make the stability rather poor, and thus they cannot be practically used.

In the techniques referred to above, because the form of formulation is restricted according to use of adjuvant components such as binder and the like, for example, an ointment condenses and becomes rigid in process of time, there is present such problem as to render the clinical use difficult. And also, in the treatment over long periods of time there is the case where it becomes necessary to use a synthetic polymer, which is not biocompatible as an adjuvant component. In the cirumstances, there is desired further improvement.

### SUMMARY OF THE INVENTION

An object of the present invention is to solve the problems raised in the prior arts as described above, and particularly is to provide a novel formulation for use in the treatment of decubitus, skin ulcer and wound, which has a high stability and biocompatibility and is capable of being prepared in the various forms of formulation without changing major adjuvant components.

The present invention provides a formulation for use in the treatment of decubitus, skin ulcer and wound, which comprises a gelatin in which a consumption of iodine is not more than 15 mg/g as measured at pH 4.5, a sugar and iodophor as essential components.

From the study by the inventors of the present invention it has been found that in the gelatins which were considered in the prior arts so that it is generally impossible to use them (Japanese Publication No. 32210/1999 B), there is present a gelatin providing a remarkable effect, when mixed with a sugar and iodophor. As the result, it has been found that there can be provided the formulation for use in the treatment of decubitus, skin ulcer and wound, which has a high stability and biocompatibility, making it possible to be prepared optionally in the various forms of formulation such as sponge, sheet, particulate or powder, ointment, and the like, and rendering possible an appropriate treatment in the stability and safety over a long period of time according to the condition of the region to be treated in a patient.

### BRIEF DESCRIPTION OF THE DRAWING

Fig. 1 is a graph showing the relation between hydrolysis time and weight avarage molecular weight for the gelatin treated with pepsin.

Fig. 2 is a graph showing the relation between hydrolysis and residual allergen activity for the gelatin treated with pepsin.

### DESCRIPTION OF THE PREFERRED EMBODIMENTS OF THE INVENTION

The gelatin used in the present invention is one in which a consumption of iodine is not more than 15 mg/g as measured at pH 4.5. Because the consumption of iodine in the normal gelatins considerably exceeds 15 mg/g, where those gelatins are employed, it is necessary to decrease the consumption of iodine beforehand. The method for decreasing the consumption of iodine is optionally selected according to the gelatin. For example, there are known the method of treating chemically gelatin with sodium hypochlorite, hydrogen peroxide, sodium borohydride or the like and the method of hydrolyzing gelatin with an acid or an enzyme to lower a molecular weight thereof. Among the gelatins obtained from young animal tissues such as bovine fetus there are present those which can satisfy the consumption of iodine, and thus those gelatins may be selected and may be used in the present invention. There is well known the method for hydrolyzing gelatin with an acid or an enzyme to lower the molecular weight thereof. For example, there are well known the hydrochloric acid degradation method in which the gelatin is hydrolyzed in the presence of hydrochloric acid and the enzyme degradation method in which the gelatin is hydrolyzed in the presence of an enzyme, but the enzyme degradation in which gelatin is hydrolyzed in the presence of pepsin is preferable (Japanese Patent Application No. 228105/1999 filed by the same application as this application). The gelatin of which the molecular weight was lowered is one which has a molecular weight of not less than 10,000, preferably not less than 20,000, most preferably about 20,000 to about 30,000.

Pepsin used in degradation (lowering of molecular weight) of gelatin may be any one of those which are commercially available. For example, there is a pepsin derived from porcine stomach mucosa, which is commercially available from Sigma. The preferable condition of degradation are as follows.

The concentration of pepsin to gelatin is such a concentration as to hydrolyze protein by using generally protease in this technical field, for example, the weight ratio of substrate to enzyme is 10,000 : 1 to 100 : 1, preferably 5,000 : 1 to 500 : 1, more preferably 1,000 : 1.

The solution is acetic acid or hydrochloric acid solution in 0.05 to 0.5 molar concentration, preferably hydrochloric acid solution in 0.1 to 0.3 molar concentration. The concentration of gelatin is 0.01 to 10%, preferably 5 to 30%. The degradation temperature is preferably 37 to 60 °C. The degradation time is preferably 1 to 12 hours, more preferably 2 to 8 hours.

The weight average molecular weight of gelatin treated with pepsin is in excess of 20,000, preferably in excess of 20,000 and up to 30,000. The pepsin-treated gelatin has allergen of not more than 1/5 of the corresponding starting gelatin and also has the ability to gelatinize.

The formulation to which the gelatin of the present invention was added provides such effects that pH of the formulation stays within a constant region, variation of pH being slight, and the effective component comprising a sugar and iodophor being stable.

In the concrete, pH of the formulation in which sucrose and povidone-iodine were mixed is within the vicinity of 1 and is very strongly acidic, and thus causes iodine and sucrose to be rapidly losed. Even if adding a pH regulator to the formualtion to neutralize it, pH value rapidly lowers in the course of time, and thereby iodine and sucrose are rapidly lost. However, it has been found that the formulation to which the gelatin of the present invention was added has pH value of 4 to 6 and provides such effects that this pH is stable over a long period of time and dose not almost lower.

The consumption of iodide in pH 4.5 in the gelatin used in the present invention is required to be not more than 15 mg/g, but it is generally preferable that the consumption of iodide is within the scope of 1 to 12 mg/g.

As a sugar, in order to maintain the stability of effective iodine in then ixed iodophor, the sugars that have no a reduction property are desirable. For example, sucrose, trehalose, gluconic acid, sorbitol, dextrin and the like are recited.

As iodophor, there are recited povidone-iodine which is a complex of iodine and polyvinyl pyrrolidone, a complex of iodine and polydextrin, and the like.

It is preferable that sugars are generally contained in an amount of 50 to 90 w/w %, preferably 60 to 80 w/w %.

It is preferable that iodophor is generally contained in an amount of 0.5 to 10 w/w %, preferably 1 to 6 w/w %.

It is preferable that the gelatin of the present invention is generally contained in an amount of 0.5 to 20 w/w %, preferably 1 to 15 w/w %, more preferably 1.5 to 5.0 w/w %.

And also, the formulation of the present invention is different from the prior art described in Japanese Patent Publication No. 32210/1989 B or the like in that the formulation of the present invention may also be prepared under the condition which is substantially free from water. Thus, it has been found that the formulation of the present invention may be prepared regardless of the presence or absence of water.

In case of necessity, the formulationof the present invention can be mixed with an optional, additional component, which is pharmaceutically acceptable. The optional components include a base agent, an additive, a solvent and the like which are generally used in pharmaceutics. For example, there are recited a hydrophilic ointment and a aborptive ointment, a water-soluble base, for example polyethylene glycol or the like; a fat and oil base, for example petrolatum, isopropyl myristate, liquid paraffin, a higher alcohol or the like; a thickener, for example acacia, alginic acid, salts thereof or the like; a water soluble polymer, for example methyl cellulose, ethyl cellulose, carboxymethyl cellulose, hydroxypropyl cellulose, hydroxypropylmethyl cellulose, polyacrylic acid, polyvinyl alcohol, salts thereof or the like; an emulsifier, for example poloxamer, polyoxyethylene alkyl ethers, polyoxyethylene hydrogenated caster oils, polysorbate, sodium lauryl sulfate, quaternary ammonium salts, hydrogenated lecithin or the like; a stabilizer, for example potassium iodide or the like; a solvent, for example a lower alcohol, glycerine, 1,3-butylene glycol, propylene glycol or the like. Water may also be used according to the form of formulation. One or more of those materials can optionally be mixed with each other. The amount of those optional components is generally not more than 40 w/w %.

In the formulation of the present invention it is not substantially necessary to adjust pH to a specific value. If necessary, however, it is possible to adjust pH by employing a pH adjuster such as hydrochloric acid, sodium hydroxide, sodium hydrogencarbonate or the like.

In addition, since in the formulation of the present invention there is mixed a gelatin, in case of necessity, it is also possible to control disintegration of the formulation and a release rate of effective components in the formulation by using a cross-linking agent such as formaldehyde, glutaraldehyde or the like, or by using transglutaminase which is an enzyme capable of cross-linking a protein.

The formulation of the present invention can be prepared in the proper form such as sponge, sheet, particulate, powder, semi-solid form or the like by using the known methods. It is also possible to microcapsulize the formulation.

Recently, the bedsore in a patient who cannot move in bed, being in a hospital for a long period of time has been raised as one of the social problems. There are various kinds in the condition of the region of bedsore, such as size of the space occurring by shear in skin, which space is called pocket. However, because the formulation of the present invention stably and safely acts over a long period of time and makes it easy to control release of the effective components and select a specific form of formulation from various forms, the formulation of the present invention is particularly useful for treatment of those bedsores.

### EXAMPLE

### Example 1

In order to clarify the action of gelatin to iodine, with regard to gelatins A to F, the experimentation of the following Test 1 was conducted.

A, B and C are an alkali-treated gelatin obtained from bone of adult bovine, and A and B are the gelatins which were further chemically treated with hydrogen peroxide.

C is the gelatin, which was obtained by treating E with pepsin (weight average molecular weight being about 20,000 to about 30,000, corresponding to the gelatin described in Japanese Patent Application No. 228105/1999 filed by the same applicant as this application).

And also, D is an alkali-treated gelatin of which the gelatin was obtained from bone of bovine fetus, F being one which was dissolved by heating a crude collagen extracted from skin of adult bovine.

The gelatins A to F did not contain impurities such as sulfurous acid.

### [Degradation of Gelatin with Pepsin]

To 100g of the alkali-treated gelatin obtained from bovine bone 300mL of purified water were added, and then heated and dissolved in the boiled water bath. After the liquid temperature was lowered to 37°C, 10g of concentrated hydrochloric acid (reagent grade, 12 N) was added and then 0.1g of pepsin (Sigma, from porcine atomach mucosa) was added with stirring, and ketp at 30°C. In other word, bovine gelatin having the concentration 25% (w/v) was hydrolyzed by treatment of pepsin (hydrochloric acid of 0.28 molar concentration, pH 3.0, pepsin 0.025%, temperature of 37 °C), and thereby the weight average molecular weight of the pepsin-treated gelatin in the treatment of pepsin within the prescribed time interval was determined. The weight average molecular weight was measured by subjecting the pepsin-treated gelatin to High-Performance Liquid Chromatography according to the method mentioned below. As a column for separation, the column in which Shodex Ohpak SB803 and Shodex Ohpak SB802. 5 (Showa Denko) were connected in series was used. As a solvent, calcium phosphate buffer (pH 6.9) was used, the flow rate being 1 mL/minute, and the temperature of the column was kept at 40°C. Molecular weight was estimated by employing polyethylene oxide molecular weight marker as a standard. For comparison, a weight average molecular weight of the gelatin degraded by treating bovine gelatin of the same concentration with hydrochloric acid (hydrochloric acid of 0.4 molar concentration, pH 1.5, temperature of 37°C) was mesured.

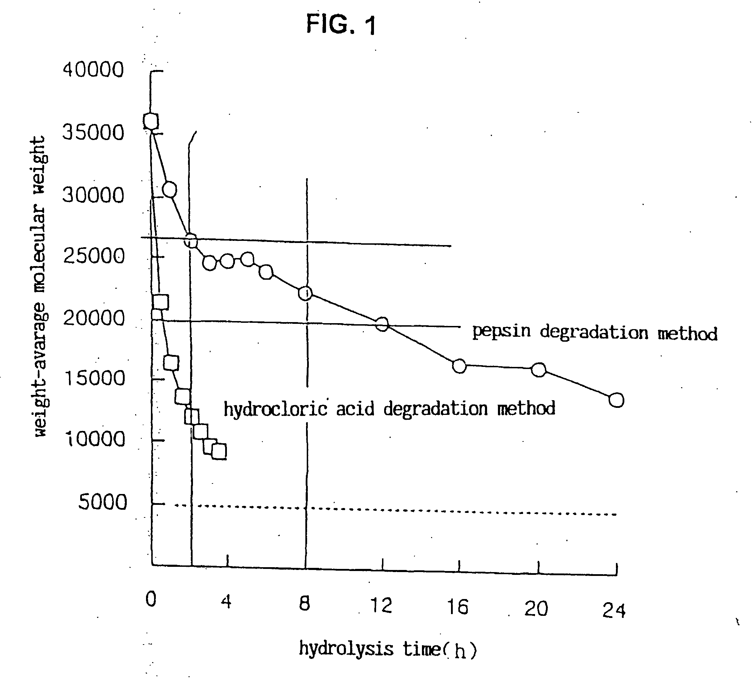

Figure 1 shows weight average molecular weight (vertical axis) to time of hydrolysis (horizontal axis).

As can be seen from figure 1, in the treatment of pepsin for 1 to 12 hours the weight average molecular weight of the degraded gelatin was not lowered to less than 20,000, whereas in the degradation with hydrochloric acid, the weight average molecular weight was lowered to about 15,000 by the treatment of 2 hours, and lowered to less than 10,000 by the treatment of 4 hours.

### [Assay of residual allergen activity in pepsin-treated gelatin]

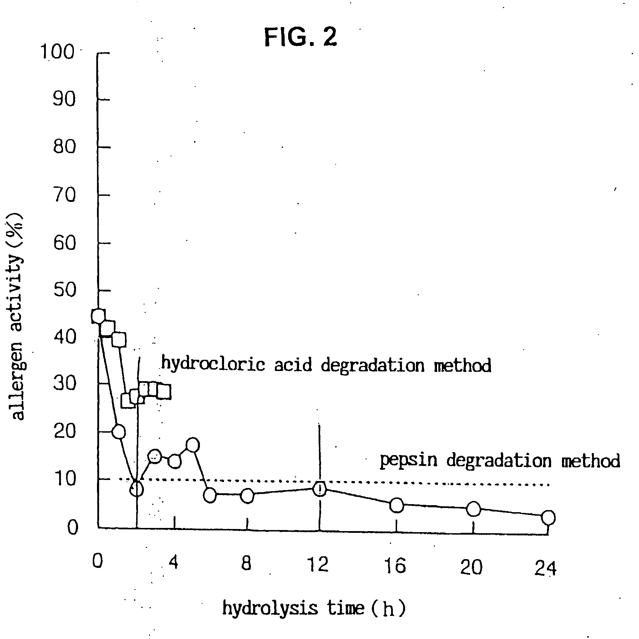

A residual allergen activity in the pepsin-treated gelatin referred to above was assayed by the fluorescence ELISA method in which serum of a patient having an allergic reaction against gelatin was used. Briefly, the pepsin-treated gelatin was absorbed into a plate for ELISA, and then IgE antibody titer of patient's serum against the gelatin was assayed by the ELISA method. The results are shown in figure 2. As shown in figure 2, the residual allergen activity in the pepsin-treated gelatin for 1 to 12 hours was lowered to less than 20%, whereas the residual allergen activity in the gelatin degraded by hydrochloric acid was merely lowered to 30%.

### [Test 1]

10mL of 0.01N iodine standard solution was added to 1% solution of each of the gelatins A to F which were adjusted to pH 4.5 with McI1vaine buffer. After allowed to stand at 50°C for 30 minutes, they were titrated with 0.005N sodium thiosulfate standard solution, and thereby the consumptions of iodine were calculated.

**[Table 1]**

| Gelatin | A | B | C | D | E | F |
|---|---|---|---|---|---|---|
| Consumption of iodine (mg/g) | 1.27 | 7.80 | 12.39 | 10.72 | 28.55 | 27.60 |

The gelatins A and B which were chemically treated, the gelatin C of which molecular weight was lowered by treatment of pepsin and the gelatin D obtained from bovine fetus were lower in the consumption of iodine than the gelatins E and F.

### Example 2

In place of the mixing amount used in Example 1, povidone-iodine, sucrose and other components were mixed in the proportion described in Table 2 and molded in the form of sheet.

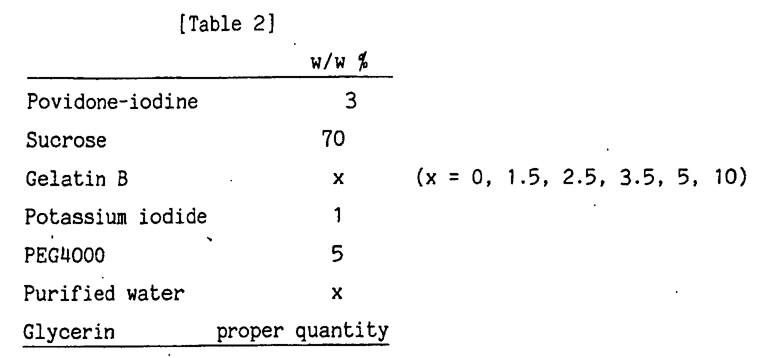

The product in which gelatin B was not mixed adhered to the film of polyethylene in the paste-like condition, when sandwiched between the films, and was not stripped away from the films. When the product was allowed to stand at a room temperature, the surface thereof was stained with liquid soon after it was prepared. In addition, even if the product was molded in the form of sheet, the sheet became liquid at the temperature of not less than 30°C and could not keep the form of sheet.

In constrast, in the products in which gelatin B was mixed the surface thereof were not stained with liquid at a room temperature. And also, the products were stripped away from the films without disintegration and kept the form of sheet.

In order to evaluate the stability of the effective components, the products were allowed to stand at 60°C for one day, and the survival rate of effective iodine after one day was calculated by measuring the content of the effective iodine. And also, pH values of the products immediately after they were prepared and one day (60 °C) after were measured, The results are shown in Table 3.

In addition, the products and a commercially available ointment containing sucrose and povidone-iodine were allowed to stand at 60°C, and variations of pH therein were compared after 14 days. The results are shown in Table 4.

**[Table 3]**

| Gelatin B (%) | | 0 | 1.5 | 2.5 | 3.5 | 5.0 | 10.0 |
|---|---|---|---|---|---|---|---|
| Survival rate (%) after one day | | ND | 98.9 | 92.9 | 95.0 | 95.1 | 92.6 |
| pH | immediately after | 1.0 | 4.2 | 4.9 | 4.9 | 5.2 | 5.1 |
| | 60 °C, One day after | 0.7 | 4.1 | 4.7 | 4.9 | 5.0 | 5.0 |
| (ND: no detection) | | | | | | | |

**[Table 4]**

| | | products gelatin B 3.5% | commercially available ointment |
|---|---|---|---|
| pH | immediately after | 4.9 | 5.6 |
| | 60 °C, one day after | 4.2 | 3.9 |

It was found from said results that the mixing amount of gelatin B greatly affected the survival rate of effective iodine and the variation of pH.

In other words, in the formulation in which gelatin B is not contained, iodine could not be detected, and the formulation molded in the form of sheet flowed in the condition of honey, and was coloured in dark brown. On the other hand, in the fromulation in which gelatin was mixed in an amount of certain range, the effective iodine was kept in the stable condition and there was not any change in appearance. And also, the variation of pH in this formulation was less than that of the commercially available ointment.

In the circumstances, it has been found that the formulation in which gelatin B was mixed improves its properties and renders the stability of the effective components higher.

### Examples 3, 4, 5, Comparative Example 1

The gelatin B or E used in Example 1 was mixed with povidone-iodine, sucrose and other components in the proportion described in Table 5, and was molded into the form of sponge or shoot. The molded products were subjected to the tests of Test 2.

### [Test 2]

Each of the samples was placed into a thermostat, and the content of effective iodine in each sample was measured by the titration method using sodium thiosulfate after 14 days to determine the survival rate thereof. The results are shown in Table 5.

**[Table 5]**

| w/w % | | | | |
|---|---|---|---|---|
| | Example 3 | Example 4 | Example 5 | Comparative Example 1 |
| Gelatin B | 3.5 | 5.0 | 2.5 | - |
| Gelatin E | - | - | - | 5.0 |
| Povidone-iodine | 3.0 | 3.0 | 3.0 | 3.0 |
| Sucrose | 70.0 | 70.0 | 70.0 | 70.0 |
| Glycerin | 21.0 | - | 16.5 | - |
| Potassium iodide | 1.0 | 1.0 | 1.0 | 1.0 |
| Formalin | 0.1 | - | - | - |
| PEG400 | - | - | 4.5 | - |
| Purified water | proper quantity | proper quantity | proper quantity | proper quantity |
| Effective iodine (Survival rate %) | 98.2 | 98.5 | 90.0 | 63.3 |

As shown in Table 5, as regards the stability of effective iodine in the sponge-like or shoot-like formulation, the formulations (Examples 3, 4, 5) in which gelatin B was used have higher stability than the formulation (Comparative Example 1) in'which gelatin B was not used.

### Examples 6, 7, Comparative Example 2

The gelatins B or E used in Example 1 was mixed with povidone-iodine, sucrose and other components in the proportion described in Table 6, and then prepared in the form of semi-solid. The products were subjected to the tests of Test 3.

### [Test 3]

Each of the samples was placed into a thermostat at 80°C, and heated for 2 hours, and then cooled to a room temperature. Thereafter, the content of effective iodine in each sample was measured by the titration method using sodium thiosulfate to determine the survival rate thereof. The results are shown in Table 6.

**[Table 6]**

| w/w % | | | |
|---|---|---|---|
| | Example 6 | Example 7 | Comparative Example 2 |
| Gelatin B | 3.5 | 5.0 | - |
| Gelatin E | - | - | 3.5 |
| Povidone-iodine | 3.0 | 3.0 | 3.0 |
| Sucrose | 70.0 | 70.0 | 70.0 |
| Potassium iodide | 1.0 | 1.0 | 1.0 |
| Glycerin | 10.0 | 10.0 | 10.0 |
| Pullulan | - | 0.2 | - |
| Purified water | proper | proper | proper |
| | quantity | quantity | quantity |
| Effective iodine ( Survival rate %) | 90.7 | 89.9 | 80.0 |

As can be seen from Table 6, in the formulation of semi-solid, the formulations (Examples 6, 7) in which gelatin B was used have higher stability of effective iodine than the formulation (Comparative Example 2) in which gelatin E was used. In addition, the stability of effective iodine was not affected by the presence or absence of pullulan.

### Examples 8, 9, Comparative Example 3, 4

The gelatin B or E used in Example 1 was mixed with povidone-iodine, sucrose and other components in the proportion described in Table 7, and dried and pulverized, and then prepared in the form of particulate or powder. The products were subjected to the tests of Test 4.

### [Test 14]

Each of the samples was placed into a thermostat at 80°C, and heated for 2 hours, and then cooled to a room temperature. Change of appearance in each of the prepared formulations was observed. The results are shown in Table 7.

**[Table 7]**

| w/w % | | | | |
|---|---|---|---|---|
| | Example 8 | Example 9 | Comparative Example | Comparative 3 Example 4 |
| Gelatin B | 4.5 | 4.8 | - | - |
| Gelatin E | - | - | 3.0 | 5.0 |
| Povidone iodine | 3.9 | 3.9 | 3.0 | 4.0 |
| Sucrose | 90.0 | 90.0 | 84.0 | 90.0 |
| Potassium iodide | 1.3 | 1.3 | - | 1.0 |
| Pullulan | 0.3 | - | - | - |
| Poloxamer | - | - | 10.0 | - |
| Before heating at 80 °C | powder | powder | powder | powder |
| After heating at 80°C | no change | no change | browning solidification | browning solidification |

As can be seem from Table 7, as to the powder, the formulations (Examples 8, 9) in which gelatin B was used maintained the conditions at the time when it was prepared even after heating, whereas the formulations (Comparative Examples 3, 4)in which gelatin E was used were browned and solidified after heating, and thus could not maintain the conditions at the time when it was prepared.

### Examples 10, 11, 12

The gelatin B or C used in Example 1 was mixed with povidone-iodine and other components in the proportion described in Table 8, and then prepared in the form of sheet without adding water at all.

The formulations were allowed to stand at 60°C for one day, and the content of effective iodine in each formulation was measured after one day to calculate the survival rate. The results are shown in Table 8.

**[Table 8]**

| w/w % | | | |
|---|---|---|---|
| | Example 10 | Example 11 | Example 12 |
| Gelatin B | 1.8 | 1.8 | - |
| Gelatin C | - | - | 1.8 |
| Povidone-iodine | 3.0 | 3.0 | 3.0 |
| Scrose | 70.0 | 70.0 | 70.0 |
| Potassium iodide | 1.0 | 1.0 | 1.0 |
| PEG400 | 5.0 | - | - |
| Purified water | - | - | - |
| Propylene glycol | - | 19.2 | - |
| Glycerin | 19.2 | - | 19.2 |
| Effective iodine (Survival rate %) | 99.5 | 99.2 | 99.2 |

Even when glycerin or propylene glycol was used in place of purified water, the formulation in which the effective iodine is stable could be prepared.

Also, even when gelatin C was mixed in place of gelatin B, the stability in the effective iodine of the same order was obtained in the prepared formulation.

### Examples 12

The gelatin B used in Example 1 was mixed with povidone-iodine, sorbitol and other components in the proportion described in Table 9 and then prepared in the form of flexible sheet

**[Table 9]**

| w/w % | |
|---|---|
| | Example 12 |
| Gelatin B | 3.5 |
| Povidone-iodine | 3.0 |
| Sorbitol | 70.0 |
| Potassium iodide | 1.0 |
| Glycerin | 10.0 |
| Purified water | proper quantity |

## Claims

1. A formulation for use in the treatment of decubitus, skin ulcer and wound, which comprises a gelatin in which a consumption of iodine is not more than 15 mg/g as measured at pH 4.5, a sugar and an iodophor as essential components.

2. The formulation according to claim 1 comprising 0.5 to 20w/w % of the gelatin, 50 to 90w/w % of the sugar, 0.5 to 10w/w % of the iodophor and, optionally, not more than 40w/w % of a pharmaceutically acceptable other component.

3. The formulation according to claim 1 or 2 which is in the form of sponge or sheet.

4. The formulation according to claim 1 or 2 which is in the form of semi-solid.

5. The formulation according to claim 1 or 2 which is in the form of particulate or powder.

6. The formulation according to claim 1 or 2 which is prepared under the conditon which is substantilly free from water.

7. The formulation according to any one of claims 1 to 6 wherein the sugar is at least one selected from the group consisting of sucrose, trehalose, gluconic acid, solbitol and dextrin.

8. The formulation according to any one of claims 1 to 6 wherein the iodophor is a complex of iodine and polyvinyl pyrrolidone or a complex of iodine and polydextrose.

9. The formulation according to any one of claims 1 to 6 wherein the pharmaceutically acceptable other component is at least one selected from the group consisting of an emulsion base, a water-soluble base, a fat and oil base, a thickener, a water-soluble polymer, an emulsifier, a stabilizer and a solvent.

10. The formulation according to any one of claims 1 to 6 wherein the gelatin is one which was chemically treated.

11. The formulation according to claim 10 wherein the gelatin is one which was chemically treated with hydrogen peroxide.

12. The formulation according to any one of claims 1 to 6 wherein the 'gelatin is one which was chemically treated with an acid or an enzyme.

13. The formulation according to claim 12 wherein the gelatin is one which was chemically treated with an enzyme.

14. The formulation according to claim 13 wherein the enzyme is pepsin.

15. The formulation according to any one of claims 12 to 14 wherein a weight average molecular weight of gelatin is not less than 10,000.

16. The formulation according to claim 15 wherein the weight average molecular weight of gelatin is not less than 20,000.

17. The formulation according to claim 15 wherein the weight average molecular weight of gelatin is 20,000 to 30,000.
